(19) Europäisches Patentamt
European Patent Office
Office européen des brevets

(11) Veröffentlichungsnummer: 0 007 421
B1

# (12) EUROPÄISCHE PATENTSCHRIFT

(45) Veröffentlichungstag der Patentschrift: 29.04.81

(21) Anmeldenummer: 79101947.4

(22) Anmeldetag: 15.06.79

(51) Int. Cl.[3]: C 07 C 69/612, A 01 N 53/00

## (54) Alpha-Isopropyl-phenylessigsäureester, ihre Herstellung und Verwendung als Schädlingsbekämpfungsmittel.

(30) Priorität: 27.06.78 CH 6991/78
12.04.79 CH 3533/79

(43) Veröffentlichungstag der Anmeldung:
06.02.80 Patentblatt 80/3

(45) Bekanntmachung des Hinweises auf die Patenterteilung
29.04.81 Patentblatt 81/17

(84) Benannte Vertragsstaaten:
BE CH DE FR GB IT NL

(56) Entgegenhaltungen:
DE - A - 2 725 767
DE - A - 2 709 264

(73) Patentinhaber: CIBA-GEIGY AG
Patentabteilung Postfach
CH-4002 Basel (CH)

(72) Erfinder: Farooq, Saleem, Dr.
Im Schaiengarten 2
CH-4107 Ettingen (CH)
Erfinder: Ackermann, Peter, Dr.
Reichensteinerstrasse 12
CH-4153 Reinach (CH)
Erfinder: Drabek, Jozef, Dr.
Benkenstrasse 12
CH-4104 Oberwil (CH)
Erfinder: Gsell, Laurenz, Dr.
Maiengasse 56
CH-4056 Basel (CH)
Erfinder: Kristiansen, Odd, Dr.
Delligrabenstrasse 7
CH-4313 Möhlin (CH)
Erfinder: Wehrli, Rudolf, Dr.
Dianastrasse 2
CH-4310 Rheinfelden (CH)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 007 421 B1

## Alpha-Isopropyl-phenylessigsäureester, ihre Herstellung und Verwendung als Schädlingsbekämpfungsmittel

Die vorliegende Erfindung betrifft Alpha-Isopropyl-phenylessigsäureester, ihre Herstellung, Schädlingsbekämpfungsmittel und ihre Verwendung zur Bekämpfung von Schädlingen.

Aus der deutschen Offenlegungsschrift No. 2 725 767 ist es bereits bekannt, dass Alpha-Isopropyl-naphthylessigsäureester insektizide und akarizide Eigenschaften haben.

Es wurde nun gefunden, dass Alpha-isopropyl-phenylessigsäureester der Formel

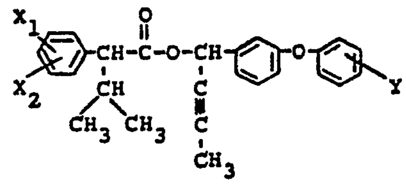

(I)

worin $X_1$ Wasserstoff, Halogen oder $C_1$—$C_4$-Alkyl,
$X_2$ Wasserstoff oder Halogen und
Y Wasserstoff, Halogen oder Methyl bedeuten,
demgegenüber ein besseres insektizides Aktivitätsspektrum aufweisen.

Unter Halogen ist dabei Fluor, Chlor, Brom oder Jod, insbesondere aber Fluor, Chlor oder Brom, zu verstehen. Die für $X_1$ in Frage kommenden Alkylgruppen sind: Methyl, Aethyl, Propyl, Isopropyl, n-, i-, sek.- oder tert.-Butyl. Wegen ihrer Wirkung von Bedeutung sind Verbindungen der Formel I, worin $X_1$ Wasserstoff, Chlor, Brom, Methyl, $X_2$ Wasserstoff oder Chlor und Y Wasserstoff, p-Fluor, p-Chlor, p-Brom oder p-Methyl bedeuten.

Insbesondere hervorzuheben sind aber Verbindungen der Formel I, worin $X_1$ Wasserstoff oder p-Chlor, $X_2$ Wasserstoff und Y Wasserstoff, p-Fluor, p-Chlor, p-Brom oder p-Methyl bedeuten.

Die Verbindungen der Formel I werden nach an sich bekannten Methoden z.B. wie folgt hergestellt:

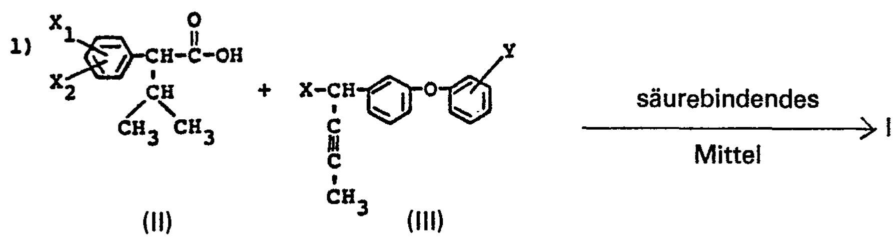


(II) (III)

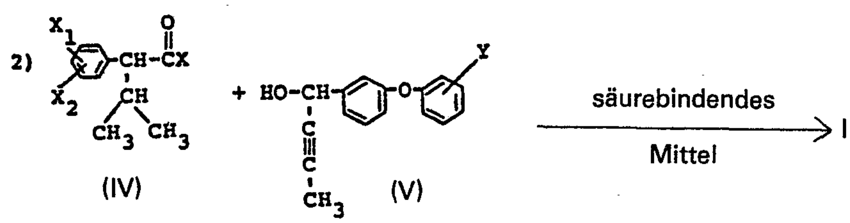


(IV) (V)

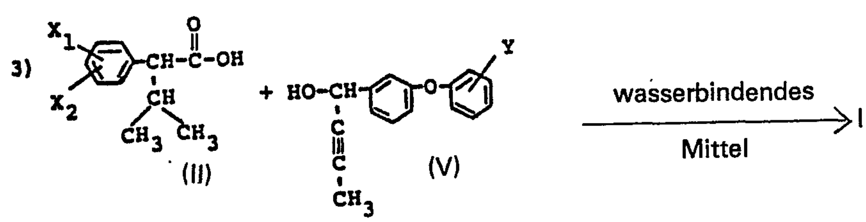


(II) (V)

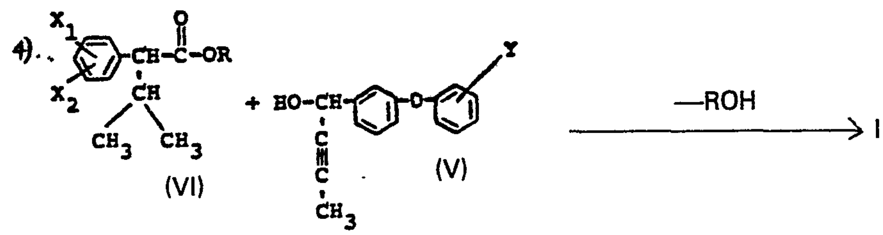


(VI) (V)

In den Formeln II bis VI haben $X_1$, $X_2$ und Y die für die Formel I angegebene Bedeutung.

In den Formeln III und IV steht X für ein Halogenatom, insbesondere Chlor oder Brom und in der Formel VI steht R für $C_1$—$C_4$-Alkyl, insbesondere für Methyl oder Aethyl. Als säurebindendes Mittel für die Verfahren 1 und 2 kommen insbesondere tertiäre Amine, wie Trialkylamin und Pyridin, ferner Hydroxide, Oxide, Carbonate und Bicarbonate von Alkali- und Erdalkalimetallen sowie Alkalimetallalkoholate wie z.B. Kalium-t.butylat und Natriummethylat in Betracht. Als wasserbindendes Mittel für das Verfahren 3 kann z.B. Dicyclohexylcarbodiimid verwendet werden. Die Verfahren 1 bis 4 werden bei einer Reaktionstemperatur zwischen —10 und 120°C, meist zwischen 20 und 80°C bei normalem oder erhöhtem Druck und vorzugsweise in einem inerten Lösungs- oder Verdünnungsmittel durchgeführt. Als Lösungsoder Verdünnungsmittel eignen sich z.B. Aether und ätherartige Verbindungen wie Diäthyläther, Dipropyläther, Dioxan, Dimethoxyäthan und Tetrahydrofuran; Amide wie N,N-dialkylierte Carbonsäureamide; aliphatische, aromatische sowie halogenierte Kohlenwasserstoffe, insbesondere Benzol, Toluol, Xylole, Chloroform und Chlorbenzol; Nitrile wie Acetonitril; Dimethylsulfoxid und Ketone wie Aceton und Methyläthylketon.

Die Ausgangsstoffe der Formeln II bis VII sind bekannt oder können analog bekannten Methoden hergestellt werden.

Die Verbindungen der Formel I liegen als Gemisch von verschiedenen optisch aktiven Isomeren vor, wenn bei der Herstellung nicht einheitlich optisch aktive Ausgangsmaterialien verwendet wurden. Die verschiedenen Isomerengemische können nach bekannten Methoden in die einzelnen Isomeren aufgetrennt werden. Unter der Verbindung der Formel I versteht man sowohl die einzelnen Isomeren, als auch deren Gemische.

Die Verbindungen der Formel I eignen sich zur Bekämpfung von verschiedenartigen tierischen und pflanzlichen Schädlingen.

Insbesondere eignen sich die Verbindungen der Formel I zur Bekämpfung von Insekten, phytopathogenen Milben und von Zecken z.B. der Ordnungen Lepidoptera, Coleoptera, Homoptera, Heteroptera, Diptera, Acarina, Thysanoptera, Orthoptera, Anoplura, Siphonaptera, Mallophaga, Thysanura, Isoptera, Psocoptera und Hymenoptera.

Vor allem eignen sich Verbindungen der Formel I zur Bekämpfung von pflanzenschädigenden Insekten, insbesondere planzenschädigenden Frassinsekten, in Zier- und Nutzpflanzen, insbesondere in Baumwollkulturen (z.B. gegen Spodoptera littoralis und Heliothis virescens) und Gemüsekulturen (z.B. gegen Leptinotarsa decemlineata und Myzus persicae).

Wirkstoffe der Formel I zeigen auch eine sehr günstige Wirkung gegen Fliegen wie z.B. Musca domestica und Mückenlarven.

Die akarizide bzw. insektizide Wirkung lässt sich durch Zusatz von anderen Insektiziden und/oder Akariziden wesentlich verbreitern und an gegebene Umstände anpassen. Als Zusätze eignen sich z.B. org. Phosphorverbindungen; Nitrophenole und deren Derivate; Formamidine; Harnstoffe; andere pyrethrinartige Verbindungen sowie Karbamate und chlorierte Kohlenwasserstoffe.

Mit besonderem Vorteil werden Verbindungen der Formel I auch mit Substanzen kombiniert, welche einen synergistischen oder verstärkenden Effekt auf Pyrethroide ausüben. Beispiele solcher Verbindungen sind u.a. Piperonylbutoxid, Propinyläther, Propinyloxime, Propinylcarbamate und Propinylphosphonate, 2-(3,4-Methylendioxyphenoxy)-3,6,9-trioxaundecan (Sesamex resp. Sesoxane), S,S,S-Tributylphosphorotrithioate, 1,2-Methylendioxy-4-(2-(octylsulfinyl)-propyl)-benzol.

Verbindungen der Formel I können für sich allein oder zusammen mit geeigneten Trägern und/oder Zuschlagstoffen eingesetzt werden. Geeignete Träger- und Zuschlagstoffe können fest oder flüssig sein und entsprechen den in der Formulierungstechnik üblichen Stoffen wie z.B. natürlichen oder regenerierten Stoffen, Lösungs-, Dispergier-, Netz-, Haft-, Verdickungs-, Binde- und/oder Düngemitteln.

Die Herstellung erfindungsgemässer Mittel erfolgt in an sich bekannter Weise durch inniges Vermischen und/oder Vermahlen der Wirkstoffe der Formel I mit den geeigneten Trägerstoffen, gegebenenfalls unter Zusatz von gegenüber den Wirkstoffen inerten Dispergieroder Lösungsmitteln. Die Wirkstoffe können in den folgenden Aufarbeitungsformen vorliegen und angewendet werden:

Feste Aufarbeitungsformen: Stäubemittel, Streumittel, Granulate (Umhüllungsgranulate, Imprägnierungsgranulate und Homogengranulate);

Flüssige Aufarbeitungsformen:

a) in Wasser dispergierbare Wirkstoffkonzentrate:
Spritzpulver (wettable powders), Pasten, Emulsionen;
b) Lösungen

Der Gehalt an Wirkstoff in den oben beschriebenen Mitteln liegt zwischen 0,1 bis 95%, dabei ist zu erwähnen, dass bei der Applikation aus dem Flugzeug oder mittels anderer geeigneter Applikationsgeräte Konzentrationen bis zu 99,5% oder sogar reiner Wirkstoff eingesetzt werden können. Die Wirkstoffe der Formel I können beispielsweise wie folgt formuliert werden (Teile bedeuten Gewichtsteile):

*Stäubemittel:* Zur Herstellung eines a) 5%igen und b) 2%igen Stäubemittels werden die folgenden Stoffe verwendet:

a) 5 Teile Wirkstoff
95 Teile Talkum;

b) 2 Teile Wirkstoff
1 Teile hochdisperse Kieselsäure
97 Teile Talkum.

Der Wirkstoff wird mit den Trägerstoffen vermischt und vermahlen.

*Granulat:* Zur Herstellung eines 5%igen Granulates werden die folgenden Stoffe verwendet:

5 Teile Wirkstoff
0,25 Teile Epichlorhydrin
0,25 Teile Cetylpolyglykoläther
3,50 Teile Polyäthylenglykol
91 Teile Kaolin (Korngrösse 0,3—0,8 mm).

Die Aktivsubstanz wird mit Epichlorhydrin vermischt und mit 6 Teilen Aceton gelöst, hierauf wird Polyäthylenglykol und Cetylpolyglykoläther zugesetzt. Die so erhaltene Lösung wird auf Kaolin aufgesprüht und anschliessend das Aceton im Vakuum verdampft.

*Spritzpulver:* Zur Herstellung eines a) 40%igen, b) und c) 25%igen, d) 10%igen Spritzpulvers werden folgende Bestandteile verwendet:

a) 40 Teile Wirkstoff
5 Teile Ligninsulfonsäure-Natriumsalz
1 Teile Dibutylnaphthalinsulfonsäure-Natriumsalz
54 Teile Kieselsäure;

b) 25 Teile Wirkstoff
4,5 Teile Calcium-Ligninsulfonat
1,9 Teile Champagne-Kreide/Hydroxyäthylcellulose Gemisch (1:1)
1,5 Teile Natrium-dibutyl-naphthalinsulfonat
19,5 Teile Kieselsäure
19,5 Teile Champagne-Kreide
28,1 Teile Kaolin;

c) 25 Teile Wirkstoff
2,5 Teile Isooctylphenoxy-polyäthylen-äthanol
1,7 Teile Champagne-Kreide/Hydroxyäthylcellulose-Gemisch (1:1)
8,3 Teile Natriumaluminiumsilikat
16,5 Teile Kieselgur
46 Teile Kaolin;

d) 10 Teile Wirkstoff
3 Teile Gemisch der Natriumsalze von gesättigten Fettalkoholsulfaten
5 Teile Naphthalinsulfonsäure/Formaldehyd-Kondensat
82 Teile Kaolin.

Der Wirkstoff wird in geeigneten Mischern mit dem Zuschlagstoff innig vermischt und auf entsprechenden Mühlen und Walzen vermahlen. Man erhält Spritzpulver, die sich mit Wasser zu Suspensionen jeder gewünschten Konzentration verdünnen lassen.

*Emulgierbare Konzentrate:* Zur Herstellung eines a) 10%igen b) 25%igen und c) 50%igen emulgierbaren Konzentrates werden folgende Stoffe verwendet:

a) 10 Teile Wirkstoff
3,4 Teile epoxydiertes Pflanzenöl
3,4 Teile eines Kombinationsemulgators, bestehend aus Fettalkoholpolyglykoläther und Alkylarylsulfonat-Calcium-Salz
40 Teile Dimethylformamid
43,2 Teile Xylol;

b) 25 Teile Wirkstoff
2,5 Teile epoxidiertes Pflanzenöl
10 Teile eines Alkylarylsulfonat/Fettalkoholpolyglykoläther-Gemisches
5 Teile Dimethylformamid
57,5 Teile Xylol;

c) 50 Teile Wirkstoff
4,2 Teile Tributylphenol-Polyglykoläther
5,8 Teile Calcium-Dodecylbenzolsulfonat
20 Teile Cyclohexanon
20 Teile Xylol.

Aus solchen Konzentraten können durch Verdünnen mit Wasser Emulsionen jeder gewünschten Konzentration hergestellt werden.

*Sprühmittel:* Zur Herstellung eines a) 5%igen und b) 95%igen Sprühmittels werden die folgenden Bestandteile verwendet:

a) 5 Teile Wirkstoff
1 Teile Epichlorhydrin
94 Teile Benzin (Siedegrenzen 160—190°C);

b) 95 Teile Wirkstoff
5 Teile Epichlorhydrin.

Beispiel 1

*a) Herstellung von α-Prop-1-ynyl-3-phenoxybenzylalkohol.*

Zu einer Lösung von 8 g Methylacetylen in 100 ml Tetrahydrofuran wird bei 0°C eine frisch aus 4 g Magnesium, 20 g Aethylenbromid in 20 ml Tetrahydrofuran hergestellte Grignardlösung langsam zugegeben und während 15 Minuten unter Argon geruhrt. Zu diesem Gemisch wird eine Lösung von 27 g o-Phenoxybenzaldehyd in 100 ml Tetrahydrofuran bei 0 bis 5°C zugetropft. Nach 14 stündigem Rühren bei Raumtemperatur wird das Reaktionsgemisch mit 50 g Eis auf 0°C abgekühlt, langsam mit 25 ml conc. Salzsäure versetzt und mit Aether extrahiert. Der Aether-Extrakt wird zweimal mit Wasser und zweimal mit gesättigter Kochsalzlösung gewaschen, über Natriumsulfat getrocknet, filtriert und eingeengt.

Das Produkt wird mit Essigester/Hexan (1:4) als Eluiermittel über Silikagel chromatographiert. Man erhält die Verbindung der Formel

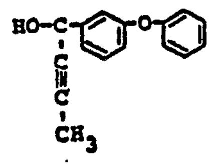

mit einem Brechungsindex von $n_D^{20°} = 1{,}5898$.

*b) Herstellung von α-Prop-1-ynyl-3-phenoxybenzyl-α-isopropyl-4-chlorphenylacetat.*

Zu einer Lösung von 3,88 g α-Isopropyl-4-chlorphenylessigsäurechlorid und 1,8 ml Pyridin in 50 ml Toluol wird bei 0°C eine Lösung von 4 g α-Prop-1-ynyl-3-phenoxybenzylalkohol in 20 ml Toluol zugetropft. Das Reaktionsgemisch wird 14 Stunden bei Raumtemperatur gerührt, dann mit Aether verdünnt, einmal mit Wasser, einmal mit 2n-Salzsäure, dreimal mit gesättigter Kochsalzlösung gewaschen über Natriumsulfat getrocknet und eingeengt. Das Produkt wird mit Aether/Hexan (1:3) als Eluiermittel über Silikagel chromatographiert. Man erhält die Verbindung der Formel

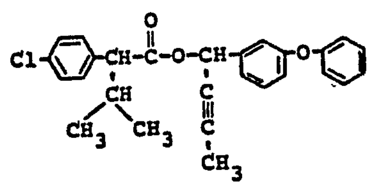

als ein diastereomeres Gemisch mit einem Brechungsindex von $n_D^{20°} = 1{,}5671$.

Auf analoge Weise werden auch folgende Verbindungen hergestellt:

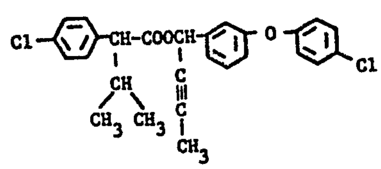

$n_D^{20°} = 1{,}5703$

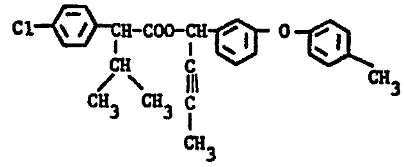

$n_D^{20°} = 1{,}5662$

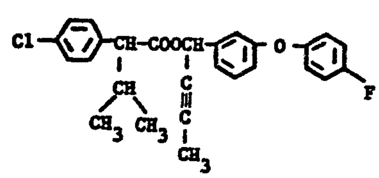

$n_D^{20°} = 1{,}5602$

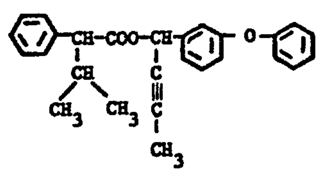

$n_D^{20°} = 1{,}5630$

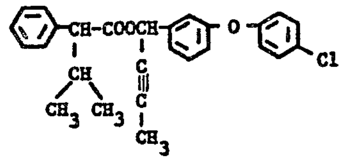

$n_D^{20°} = 1,5650$

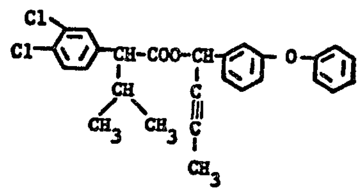

$n_D^{20°} = 1,5771$

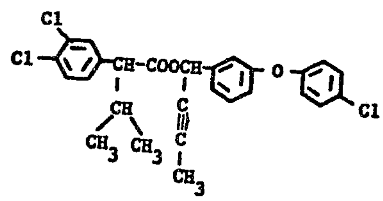

$n_D^{20°} = 1,5792$

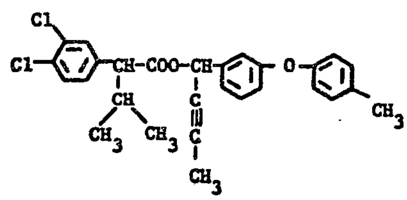

$n_D^{20°} = 1,5718$

## Beispiel 2

A) *Insektizide Frassgift-Wirkung*

Baumwollpflanzen wurden mit einer 0,05%igen wässerigen Wirkstoffemulsion (erhalten aus einem 10%igen emulgierbaren Konzentrat) besprüht.

Nach dem Antrocknen des Belages wurden die Baumwollpflanzen je mit Spodoptera littoralis- und Heliothis virescens-Larven $L_3$ besetzt. Der Versuch wurde bei 24°C und 60% relativer Luftfeuchtigkeit durchgeführt.

Verbindungen gemäss Beispiel 1 zeigten im obigen Test eine gute insektizide Frassgift-Wirkung gegen Spodoptera- und Heliothis-Larven.

## Beispiel 3

*Akarizide Wirkung*

Phaseolus vulgaris Pflanzen wurden 12 Stunden vor dem Test auf akarizide Wirkung mit einem infestierten Blattstück aus einer Massensucht von Tetranychus urticae belegt. Die übergelaufenen beweglichen Stadien wurden aus einem Chromatographierzerstäuber mit den emulgierten Testpräparaten derart besprüht, dass kein Ablaufen der Spritzbrühe eintrat. Nach zwei und 7 Tagen wurden Larven, Adulte und Eier unter dem Binokular auf lebende und tote Individuen ausgewertet und das Ergebnis in Prozenten ausgedrückt. Während der "Haltezeit" standen die behandelten Pflanzen in Gewächshauskabinen bei 25°C. Verbindungen gemäss Beispiel 1 wirkten im obigen Test gegen Adulte, Larven und Eier von Tetranychus urticae.

## Beispiel 4

*Wirkung gegen Zecken*

A) Rhipicephalus bursa

Je 5 adulte Zecken bzw. 50 Zeckenlarven wurden in ein Glasröhrchen gezählt und für 1 bis 2 Minuten in 2 ml einer wässrigen Emulsion aus einer Verdünnungsreihe mit je 100, 10, 1 oder 0,1 ppm Testsubstanz getaucht. Das Röhrchen wurde dann mit einem genormten Wattebausch verschlossen und auf den Kopf gestellt, damit die Wirkstoffemulsion von der Watte aufgenommen werden konnte.

Die Auswertung erfolgte bei den Adulten nach 2 Wochen und bei den Larven nach 2 Tagen. Für jeden Versuch liefen 2 Wiederholungen.

B) Boophilus microplus (Larven)

Mit einer analogen Verdünnungsreihe wie beim Test A) wurden mit je 20 sensiblen resp. OPresistenten Larven Versuche durchgeführt. (Die Resistenz bezieht sich auf die Verträglichkeit von Diazonion). Verbindungen gemäss Beispiel 1 wirkten in diesen Tests gegen Adulte und Larven von Rhipicephalus bursa und sensible resp. OP-resistente Larven von Boophilus microplus.

**Patentansprüche**

1. Alpha-isopropyl-phenylessigsäureester der Formel

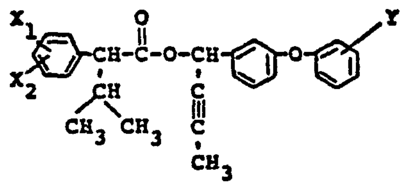

worin $X_1$ Wasserstoff, Halogen oder $C_1$—$C_4$-Alkyl,
$X_2$ Wasserstoff oder Halogen und
Y Wasserstoff, Halogen oder Methyl bedeuten.

2. Alpha-Isopropyl-phenylessigsäureester gemäss Anspruch 1,
worin $X_1$ Wasserstoff, Chlor, Brom, Methyl,
$X_2$ Wasserstoff oder Chlor und
Y Wasserstoff, p-Fluor, p-Chlor, p-Brom oder p-Methyl bedeuten.

3. Alpha-Isopropyl-phenylessigsäureester gemäss Anspruch 1,
worin $X_1$ Wasserstoff oder p-Chlor
$X_2$ Wasserstoff und
Y Wasserstoff, p-Fluor, p-Chlor, p-Brom oder p-Methyl bedeuten.

4. Der Alpha-Isopropyl-phenylessigsäureester gemäss Anspruch 1 der Formel

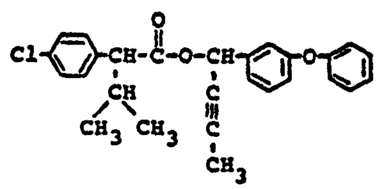

5. Der Alpha-Isopropyl-phenylessigsäureester gemäss Anspruch 1, der Formel

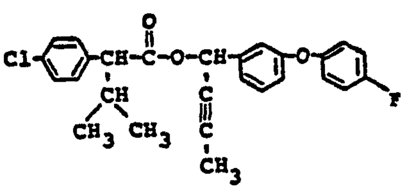

6. Verfahren zur Herstellung einer Verbindung gemäss Anspruch 1, dadurch gekennzeichnet, dass man eine Verbindung der Formel

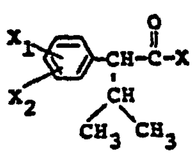

in Gegenwart eines säurebindenden Mittels mit einer Verbindung der Formel

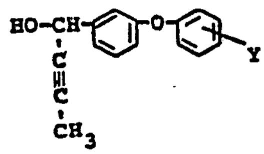

umsetzt, worin $X_1$, $X_2$ und Y die im Anspruch 1 angegebene Bedeutung haben und X für ein Halogenatom steht.

7. Schädlingsbekämpfungsmittel, welches als aktive Komponente eine Verbindung gemäss Anspruch 1 und geeignete Träger- und/oder andere Zuschlagstoffe enthält.

8. Verwendung einer Verbindung gemäss Anspruch 1 zur Bekämpfung von tierischen und pflanzlichen Schädlingen.

9. Verwendung gemäss Anspruch 8 zur Bekämpfung von Insekten und Vertretern der Ordnung Akarina.

## Claims

1. An $\alpha$-isopropylphenylacetate of the formula

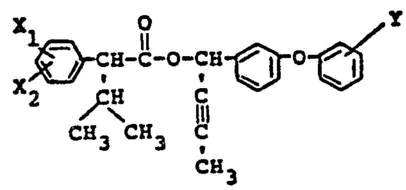

wherein $X_1$ represents hydrogen, halogen or $C_1$—$C_4$alkyl, $X_2$ represents hydrogen or halogen, and Y represents hydrogen, halogen or methyl.

2. An $\alpha$-isopropylphenylacetate according to claim 1, wherein $X_1$ represents hydrogen, chlorine, bromine or methyl, $X_2$ represents hydrogen or chlorine, and Y represents hydrogen, p-fluorine, p-chlorine, p-bromine or p-methyl.

3. An $\alpha$-isopropylphenylacetate according to claim 1, wherein $X_1$ represents hydrogen or p-chlorine, $X_2$ represents hydrogen, and Y represents hydrogen, p-fluorine, p-chlorine, p-bromine or p-methyl.

4. The $\alpha$-isopropylphenylacetate according to claim 1 of the formula

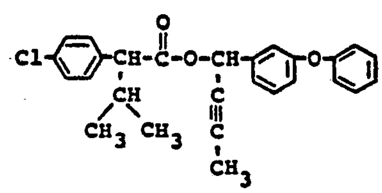

5. The $\alpha$-isopropylphenylacetate according to claim 1 of the formula

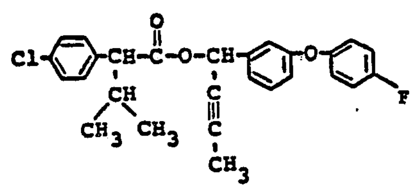

6. A process for the manufacture of a compound according to claim 1, characterised in that a compound of the formula

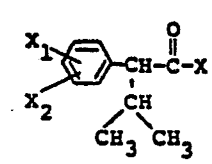

is reacted, in the presence of an acid acceptor, with a compound of the formula

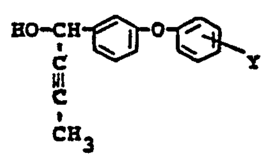

wherein $X_1$, $X_2$ and Y are as defined in claim 1 and X represents a halogen atom.

7. A pesticidal composition which contains, as active component, a compound according to claim 1 and suitable carriers and/or other adjuvants.

8. A method of controlling animal and plant pests which comprises the use of a compound according to claim 1.

9. A method according to claim 8, wherein the pests to be controlled are insects and representatives of the order Acarina.

**Revendications**

1. Esters alpha-isopropyl-phénylacétiques de formule

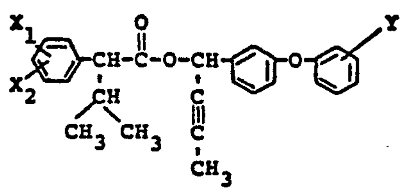

dans laquelle:
— $X_1$ représente l'hydrogène, un halogène ou un groupe alkyle en C1—C4,
— $X_2$ représente l'hydrogène ou un halogène et
— Y représente l'hydrogène, un halogène ou un groupe méthyle.

2. Esters alpha-isopropyl-phénylacétiques selon la revendication 1, dans lesquels:
— $X_1$ représente l'hydrogène, le chlore, le brome, un groupe méthyle,
— $X_2$ représente l'hydrogène ou le chlore et
— Y représente l'hydrogène, le fluor, le chlore ou le brome en position para ou un groupe méthyle en position para.

3. Esters alpha-isopropyl-phénylacétiques selon la revendication 1, dans lesquels:
— $X_1$ représente l'hydrogène ou le chlore en position para,
— $X_2$ représente l'hydrogène et
— Y représente l'hydrogène, le fluor, le chlore ou le brome en position para, ou un groupe méthyle en position para.

4. L'ester alpha-isopropyl-phénylacétique selon la revendication 1, de formule

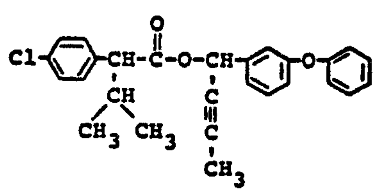

5. L'ester alpha-isopropyl-phénylacétique selon la revendication 1, de formule

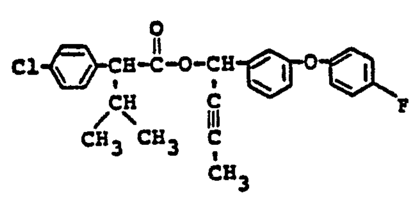

6. Procédé de préparation d'un composé selon la revendication 1, caractérisé en ce que l'on fait réagir un composé de formule

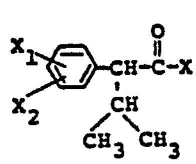

en présence d'un agent fixant les acides, avec un composé de formule

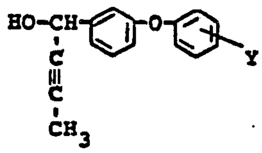

les symboles $X_1$, $X_2$ et Y ayant les significations indiquées dans la revendication 1, et X représentant un atome d'halogène.

7. Pesticides contenant en tant que composant actif un composé selon la revendication 1 et des véhicules et/ou autres additifs appropriés.

8. Utilisation d'un composé selon la revendication 1, dans la lutte contre les parasites animaux et végétaux.

9. Utilisation selon la revendication 8, dans la lutte contre les insectes et les représentants de l'ordre des acariens.